# EUROPEAN PATENT SPECIFICATION

(11) **EP 1 986 985 B1**
(45) Date of publication and mention of the grant of the patent: **01.09.2010**
(21) Application number: 06832040.7
(22) Date of filing: 01.12.2006
(51) Int. Cl.: C07C 51/08, C07C 43/23, C07C 255/54, C07C 309/65

(54) **INTERMEDIATES AND PROCESS FOR THE PREPARATION OF AROMATIC DERIVATIVES OF 1-ADAMANTANE**
ZWISCHENPRODUKTE UND VERFAHREN ZUR HERSTELLUNG AROMATISCHER DERIVATE VON 1-ADAMANTAN
INTERMEDIAIRES ET PROCEDE DE PREPARATION DE DERIVES AROMATIQUES DE 1-ADAMANTANE

(30) Priority: 02.12.2005 ES 200503057
(43) Date of publication of application: 05.11.2008
(73) Proprietor: FINORGA SAS, 38670 Chasse sur Rhône (FR)
(72) Inventor: COMELY, Alexander Christian, E-08028 Barcelona (ES); MARFIL SÁNCHEZ, Marta, E-08028 Barcelona (ES); RAFECAS JANÉ, Llorenç, E-08028 Barcelona (ES); RIERA ESCALÉ, Antoni, E-08028 Barcelona (ES)
(74) Representative: Hirsch & Associés
(86) International application number: PCT/IB2006/054548
(87) International publication number: WO 2007/063523

(56) References cited:
- EP-A1- 0 199 636
- WO-A-2005/108338
- WO-A-2006/108717
- WO-A-2007/028104

## Description

The present invention refers to the obtaining of aromatic derivatives of 1-adamantane, in particular of Adapalene, as well as intermediate compounds for its preparation.

### BACKGROUND ART

Adapalene is the International Non-proprietary Name (INN) of the active pharmaceutical ingredient, the chemical name of which is 6-[3-(1-(adamantyl)-4-methoxyphenyl]-2-naphthoic acid, and which has the formula:

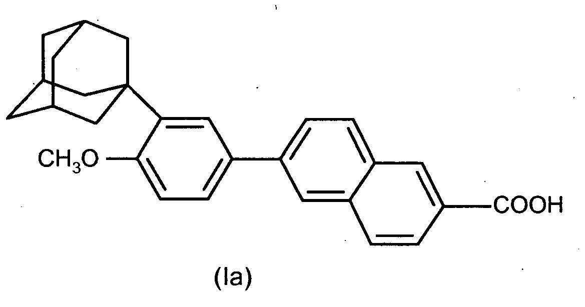

Adapalene is an antiacne agent, derived from naphthoic acid, with antiinflammatory and keratolytic properties.

Patent application EP 199.636-A1 describes benzonaphthalenic derivatives and their therapeutic and cosmetic utility. It also describes a process for their preparation. Among the compounds described are various derivatives of 1-adamantane, such as Adapalene, that is obtained by the transformation of 2-(adamantyl)-4-halogen anisole into its magnesium, lithium or zinc derivative, followed by coupling with methyl 6-bromo-2-naphthoate and subsequent hydrolysis of the ester obtained in basic conditions. This process presents the drawback that the halogenated derivatives of methyl naphthoate are hard to prepare and are obtained with low yields.

On the other hand, patent application WO 01/56563-A1 describes various aromatic derivatives of 1-adamantane, including Adapalene, and their use for the treatment and/or prevention of cancer.

Thus, based on what is known in the state of the art, it can be derived that the provision of an alternative process for the preparation of aromatic derivatives of 1-adamantane, that were efficient and of easy industrialization, would be of great interest for the industrial manufacture of this type of compound.

### SUMMARY OF THE INVENTION

The inventors have found a new, easy and simple process for the preparation of phenylnaphtalene-carboxylic derivatives of 1-adamantane, based on obtaining, at the end of the synthesis, the carboxylic group from the corresponding cyano derivative. In particular, the process is especially advantageous for the preparation of Adapalene, because it does not require the preparation of naphthalene-carboxylic compounds, which are intermediates that are expensive and hard to prepare.

Thus, according to an aspect of the present invention, a compound of formula (II) is provided, 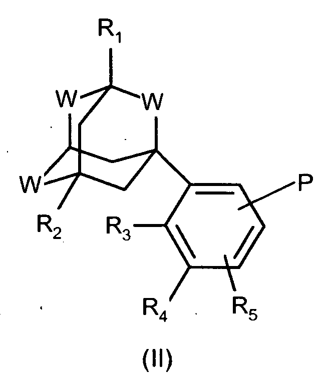 where W is a biradical selected from the group consisting of: -CH₂-, -O- and--SO₂-; R₁ and R₂ are radicals, equal or distinct, independently selected from the group consisting of H, halogen and (C₁-C₆)-alkyl ; R₃ is a radical selected from the group consisting of hydroxyl, acyl, amide, halogen, (C₁-C₆)- alkyl optionally substituted for one or more hydroxyl or acyl groups, and (C₁-C₄)-alkoxy optionally substituted by one or more hydroxyl, (C₁-C₄)-alkoxy or amide groups, and/or optionally interrupted by one or more oxygen atoms; R₄ is a radical selected from the group consisting of H, hydroxyl, (C₁-C₆)-alkyl, and (C₁-C₄)-alkoxy; or R₃ and R₄ together form a biradical -OCH₂O-; R₅ is a radical selected from the group consisting of H, (C₁-C₆)-alkyl, (C₁-C₄)-alkoxy, and a halogen; and P is a radical selected from (P)-1 and (P)-2: 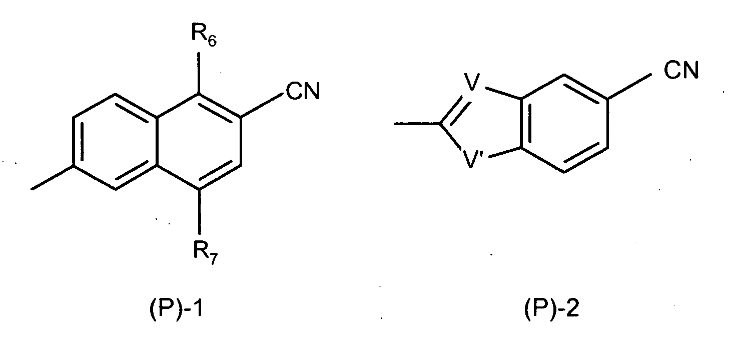 where R₆ is a radical selected from H, (C₁-C₆)-alkyl and halogen; R₇ is a radical selected from H, hydroxyl and halogen; V is a biradical -CH- and V' is an O atom; or V is an N atom and V' is a biradical -NH-.

In a preferred embodiment, the compound of formula (II) is that compound where W is CH₂. In another more preferred embodiment the compound of formula (II) is that where P is the radical (P)-1. The most preferred compound is 6-[3-(1-adamantyl)-4-methoxyphenyl]-2-cyanonaphthalene of formula (IIa):

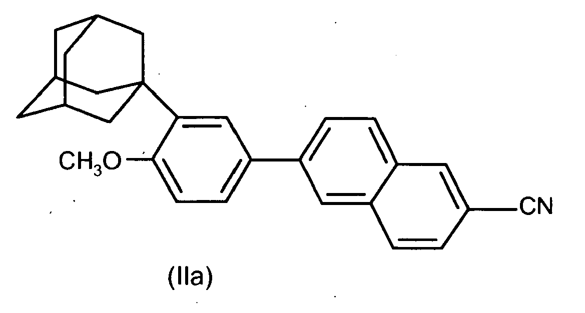

According to another aspect of the present invention, a process is provided for the preparation of the compound of formula (II) as defined previously that comprises reacting a compound of formula (III) with a metal cyanide, preferably selected from copper and zinc.

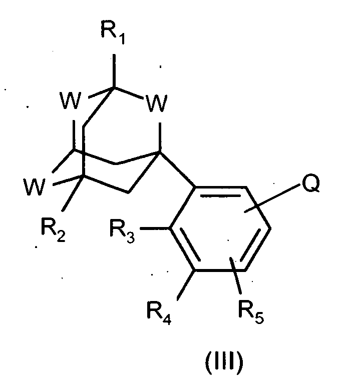

In formula (III), Q is a radical selected from the following: 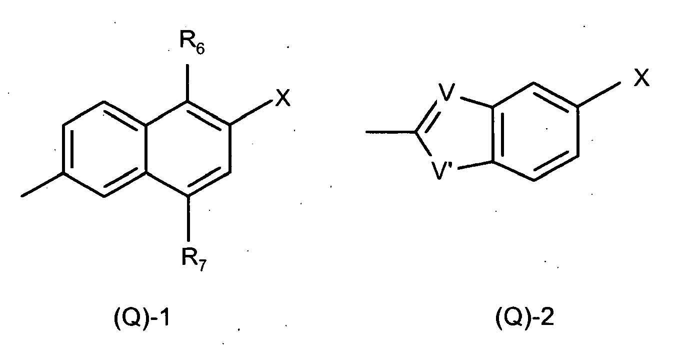 where X is a leaving group selected from a halogen such as Cl, Br and I, and a sulfonate of formula -OSO₂R₉ where R₉ is selected from the group consisting of CF₃, (C₁-C₄)-alkyl, phenyl, and phenyl that is mono- or disubstituted by a radical selected from (C₁-C₄)-alkyl, halogen and nitro; and W, V, V', R₁, R₂, R₃, R₄, R₅, R₆ and R₇ have the same meaning as defined above for the compound of formula (II).

In a preferred embodiment the compound of formula (III) is the compound of formula (IIIa) where X has the same meaning as that in (III). 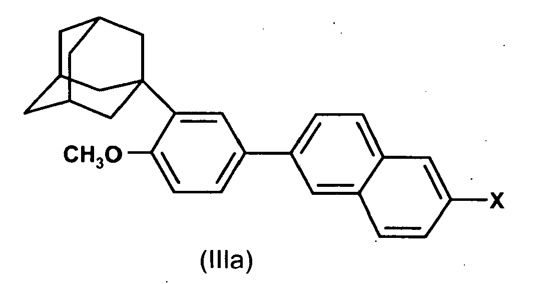 Preferably, the halogen is Br and the sulfonate is selected from mesylate (R₉= -CH₃), tosylate (R₉= -C₆H₄CH₃), besylate (R₉= -C₆H₅) and trifluoromethanesulfonate (R₉= -CF₃), and more preferably is trifluoromethanesulfonate.

In a preferred embodiment, the cyanation of the compound of formula (IIIa) in which X is Br is carried out with copper cyanide. In another preferred embodiment, the cyanation of compounds of formula (IIIa), in which X is trifluoromethanesulfonate, is carried out with zinc cyanide, preferably in the presence of a palladium or nickel catalyst.

The compounds of formula (III) where X is a halogen can be obtained by a coupling reaction between a compound of formula (IV) or a compound of formula (V) 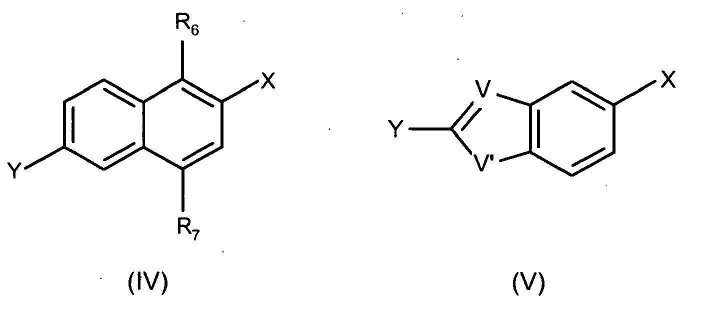 and, either 2,4,6-tris[3-(1-adamantyl)-4-methoxyphenyl]-1,3,5,2,4,6-trioxatriborinane, or a compound of formula (VI) 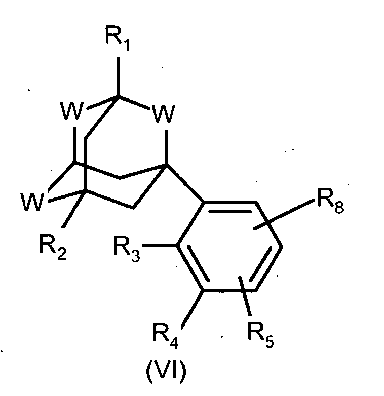 where R₈ is a radical selected from MgZ, ZnZ and a radical of the formula 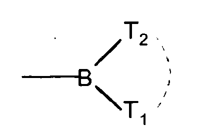 where Z is a halogen, preferably Cl or Br, and T₁ and T₂ are each independently selected from the group consisting of hydroxyl, (C₁-C₄)-alkoxy and phenoxyl, the latter optionally substituted by a (C₁-C₄)-alkoxy, (C₁-C₄)-alkyl or halogen group; or alternatively T₁ and T₂ are taken together with the boron atom to form a cyclic structure selected from the following, 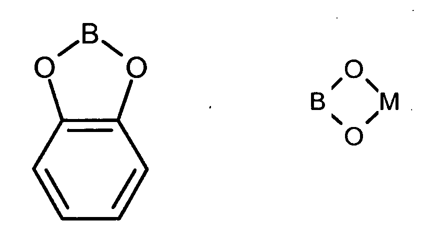 where M is selected from the group consisting of (CH₂)ₙ, (CH₂)ᵣCRᵤRᵥ(CH₂)ₛ and CRᵤRᵥ(CH₂)ₜCRᵤRᵥ; n is an integer from 2 to 4; r and s are integers from 0 to 4 with the condition that r and s are not both 0; t is an integer from 0 to 1, and Rᵤ and Rᵥ are each independently selected from the group consisting of H, (C₁-C₄)-alkyl, phenyl and mono- or disubstituted phenyl, with the substituents being halogen, (C₁-C₄)-alkyl and (C₁-C₄)-alkoxy.

In the case of the derivatives of Zn, the reaction is known as the Negishi coupling. In the case of the derivatives of boron, this reaction is known as the Suzuki coupling. Generally, these reactions are carried out in the presence of an adequate solvent and preferably, in the presence of a transitional metal compound. The Suzuki coupling is preferably carried out in the presence of a base.

In the formulas (IV), (V) and (VI), W, R₁, R₂, R₃, R₄, R₅, R₆ and R₇ have the same meaning as that defined above for the compound of formula (II); X is a halogen selected from Cl, Br and I, and Y represents a leaving group, selected from a halogen such as Cl, Br and I, and a sulfonate of formula -OSO₂R₉ that is selected from the group consisting of CF₃, (C₁-C₄)-alkyl such as methyl, phenyl;and phenyl that is mono- or disubstituted by a radical selected from (C₁-C₄)-alkyl such as methylphenyl, halogen, and nitro.

As an example, the compound of formula (IIIa) where X is a halogen, is obtained by coupling between a compound of formula (IVa), i.e., compound (IV) with R₆ and R₇=H and X=halogen and, either 2,4,6-tris[3-(1-adamantyl)-4-methoxyphenyl]-1,3,5,2,4,6-trioxatriborinane or a compound of formula (VIa);

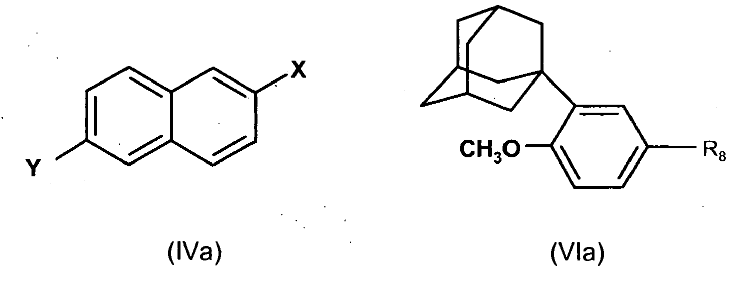

In the formulae (IVa) and (VIa), R₈ and Y have the same meaning as described above.

Preferably, to obtain the compound of formula (IIIa) with X= Br, the compound of formula (IVa) with Y= Br or trifluoromethanesulfonate and X= Br, is coupled with 2,4,6-tris[3-(1-adamantyl)-4-methoxyphenyl]-1,3,5,2,4,6-trioxatriborinane, or with a compound of formula (VIa) selected from
3-(1-adamantyl)-4-methoxyphenylboronic acid,
(3-(1-adamantyl)-4-methoxyphenyl)-5,5-dimethyl-1,3,2-dioxaborinane, and
3-(1-adamantyl)-4-methoxybenzene zinc chloride.

The compound of formula (IVa) in which X and Y are Br, i.e., 2,6-dibromonaphthalene is commercially available. The compound of formula (IVa) in which X is Br and Y is a radical trifluoromethanesulfonate (i.e., trifluoromethanesulfonate of 6-bromo-2-naphthalene) can easily be prepared from 6-bromo-2-naphthol by reaction with triflic anhydride, generally in the presence of a tertiary amide, such as triethylamine.

As an example, the lithiation of the commercial product 3-(1-adamantyl)-1-bromo-4-methoxybenzene, followed by treatment with triisopropyl borate (B(O-i-Pr)₃) and acid hydrolysis, leads to impure 3-(1-adamantyl)-4-methoxyphenylboronic acid in the form of free boronic acid. This compound can be converted into the trimeric cycled product 2,4,6-tris[3-(1-adamantyl)-4-methoxyphenyl]-1,3,5,2,4,6-trioxatriborinane, by heating at about 60 °C or by treating with a (C₆-C₈)-aliphatic hydrocarbon such as hexane, which can be carried out even a low temperatures (0-5 °C).

The preparation of other boronic derivatives of formula (VIa) such as (3-(1-adamantyl)-4-methoxyphenyl)-5,5-dimethyl-1,3,2-dioxaborinane, can, for example, be carried out via the reaction of the trimeric product, 2,4,6-trips[3-(1-adamantyl)-4-methoxyphenyl]-1,3,5,2,4,6-trioxatriborinane with 2,2-dimethyl-1,3-propandiol in an appropriate solvent such as a (C₆-C₈)-aromatic hydrocarbon and at high temperatures.

The preparation of zinc derivatives of formula (VIa) such as 3-(1-adamantyl)-4-methoxybenzene zinc chloride, can, for example, be carried out by treatment of the lithium or magnesium derivative of 3-(1-adamantyl)-1-bromo-4-methoxybenzene with ZnCl₂.

Preferably, the transition metal compound used to carry out the coupling between a compound of (IV) or (V) and a compound of formula (VI) leading to the compound of formula (III), is selected from the metal salts and metal complexes of palladium and nickel. Examples of appropriate metal compounds are: tetrakis(triphenylphosphine)palladium (0), (Pd(PPh₃)₄); [1,1'-bis(diphenylphosphino)ferrocene]dichloropalladium (II), (PdCl₂(dppf)); dichloro[1,4-bis(diphenylphosphino)butane]palladium, (PdCl₂(dppb)); dichlorobis(tricyclohexylphosphine)palladium (II), (PdCl₂(PCy₃)₂); dichloro[1,1'-bis(di-tert-butylphosphino)ferrocene]palladium (II), (PdCl₂(dtbp)); palladium; palladium chloride; palladium acetate; dichlorobis(triphenylphosphine)nickel (II), NiCl₂(PPh₃)₂; and mixtures of previously mentioned catalysts with phosphines. Preferably, the metal compound is tetrakis(triphenylphosphine)palladium (0), (Pd(PPh₃)₄).

Preferably the coupling is carried out at a temperature comprised between room temperature and the reflux temperature of the solvent used. Preferably, the base used for the Suzuki coupling is selected from a metal alkaline carbonate and a metal alkaline phosphate. More preferably, the base is potassium phosphate.

The compounds of formula (III) where X is a radical of sulfonate, can be prepared by reaction of the corresponding alcohol with a sulfonyl chloride or a anhydride of sulfonic acid. Preferably, triflic anhydride is used. The reaction is carried out in an appropriate solvent in the presence of a tertiary amine and at a temperature comprised between approximately -15 °C and 30 °C. Appropriate solvents to carry out the reaction are, for example, chloride solvents such as dichloromethane or 1,2-dichloroethane. Examples of appropriate tertiary amines are triethylamine or diisopropylethylamine.

The precursor alcohol can be obtained by coupling of a compound of formula (VII) or a compound of formula (VIII) and, either 2,4,6-tris[3-(1-adamantyl)-4-methoxyphenyl]-1,3,5,2,4,6-trioxatriborinane or a compound of formula (VIb). In general, it is carried out in the presence of an adequate solvent and, preferably, in the presence of a transition metal compound and a base.

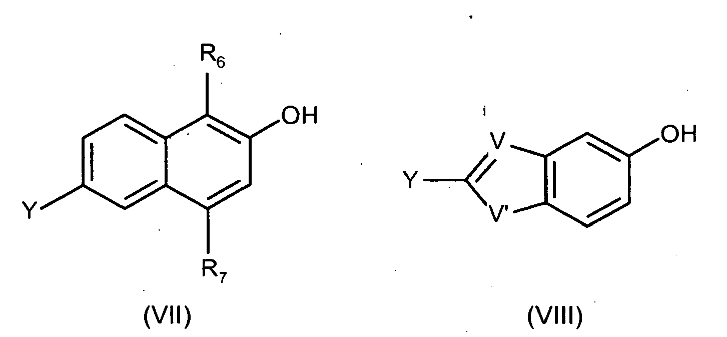

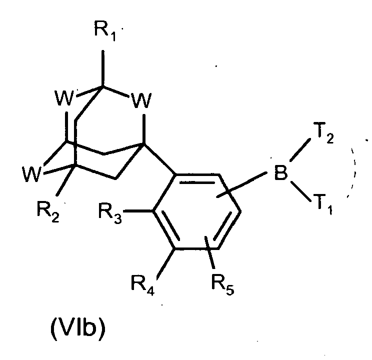

In the above formulae, all of the variables and substituents have the same meaning as those defined previously for compounds (IV), (V) and (VI).

The reaction conditions in which the coupling between compounds (VII) and (VIII) and the compound (VI) is carried out are basically the same as those for the coupling of a compound of formula (IV) or (V) and the compound (Vlb) described above.

By way of example, the alcohol of formula (IX) is obtained by coupling between a compound of formula (VIIa), i.e., compound (VII) with R₆ and R₇=H, and either 2,4,6-tris[3-(1-adamantyl)-4-methoxyphenyl]-1,3,5,2,4,6-trioxatriborinane or a compound of formula (Vlc), i.e., compound (VIa) where R₈ is a derivative of boron.

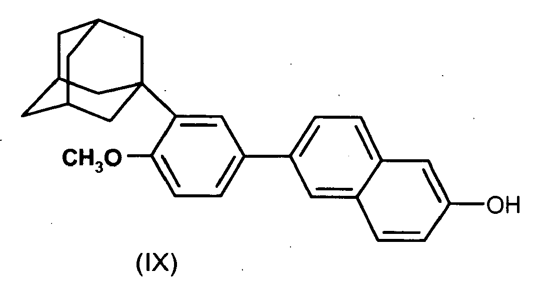

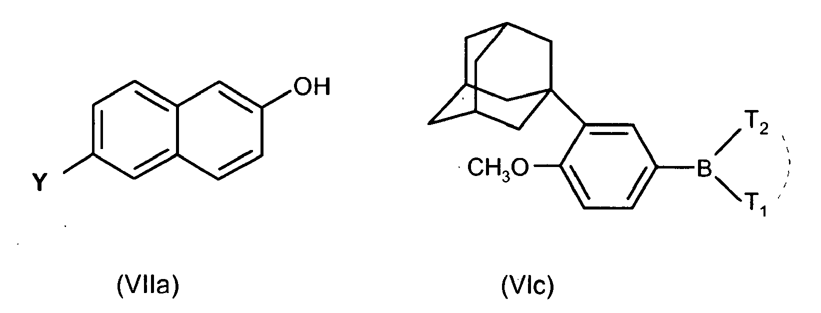

In the formula (VIIa), Y has the same meaning as that described previously for the compound of formula (VII) and T₁ and T₂ have the same meaning as in (VIb).

The compounds of formula (IIIa) where X is a sulfonate radical, that is compound (X), where R₉ is selected from CF₃, (C₁-C₄)-alkyl, phenyl, and phenyl that is mono- or disubstituted by a radical selected from (C₁-C₄)-alkyl, halogen and nitro, and the precursor alcohol of the same of formula (IX), are new compounds that also form part of the present invention. In a preferred embodiment, the compound of formula (X) is that where R₉ is CF₃.

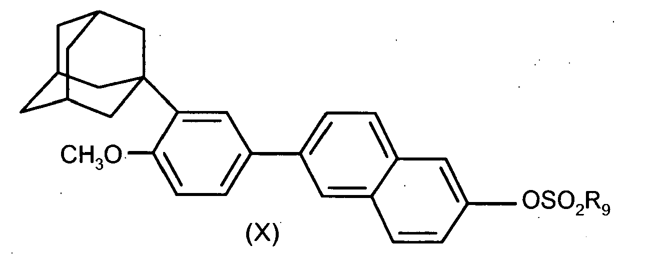

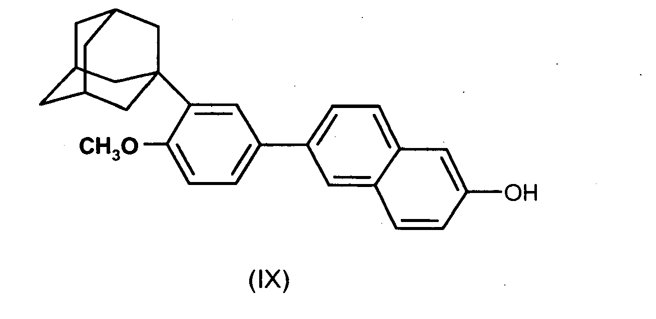

Also forming part of the present invention is an alternative preparation process of a compound of formula (II), that comprises a coupling reaction between the compound (XI) or the compound (XII) 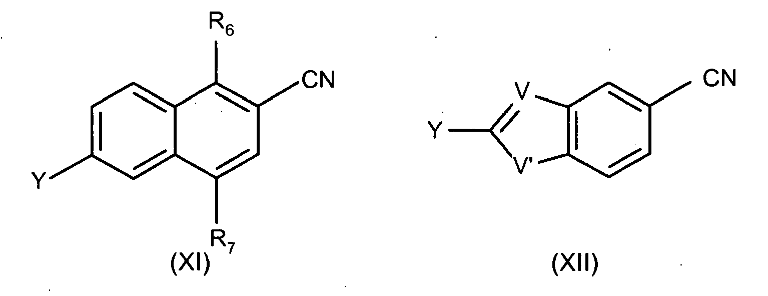 and, either 2,4,6-tris[3-(1-adamantyl)-4-methoxyphenyl]-1,3,5,2,4,6-trioxatriborinane, or a compound of formula (VI); 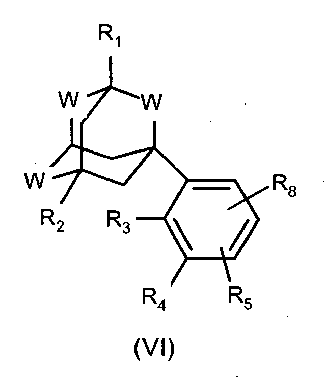 where: Y, V, V', W, R₁, R₂, R₃, R₄, R₅, R₆, R₇ and R₈ have the same meaning as that defined above. Preferably, Y is selected from Br, methansulfonate, p-toluensulfonate and trifluoromethanesulfonate.

In a preferred embodiment, the coupling reaction is carried out with the compound of formula (Xla), i.e. compound (XI) in which R₆ and R₇ = H and Y has the same meaning as in compound (XI).

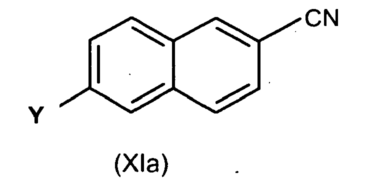

In a more preferred embodiment, in the compound of formula (XIa), Y is selected from Br, methansulfonate, p-toluensulfonate and trifluoromethanesulfonate.

In another preferred embodiment, the compound (VI) is the compound of formula (VIa) where R₈ has the same meaning as in (VI).

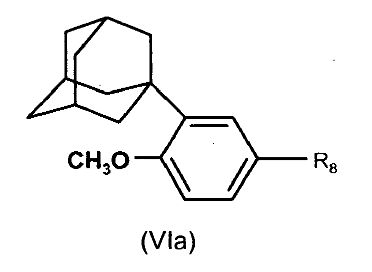

Preferably the compound of formula (Xla) is coupled with 2,4,6-tris[3-(1-adamantyl)-4-methoxyphenyl]-1,3,5,2,4,6-trioxatriborinane, or with a compound of formula (VIa) selected from 3-(1-adamantyl)-4-methoxyphenylboronic acid, (3-(1-adamantyl)-4-methoxyphenyl)-5,5-dimethyl-1,3,2-dioxaborinane, 3-(1-adamantyl)-4-methoxybenzene zinc chloride, 3-(1-adamantyl)-4-methoxybenzene zinc bromide and 3-(1-adamantyl)-4-methoxybenzene magnesium bromide.

Generally, the coupling reaction between a compound of formula (XI) or (XII) and a compound of formula (VI) leading to the compound of formula (II) is carried out in the presence of an appropriate solvent and, preferably, in the presence of a transition metal compound. In the event that boron derivatives are used, it should preferably be done in the presence of a base.

Preferably, the metal compound is selected from the metal salts and metal complexes of palladium and nickel. Examples of appropriate metal compounds are: tetrakis(triphenylphosphine)palladium (0), (Pd(PPh₃)₄); [1,1'-bis(diphenylphosphino)ferrocene]dichloropalladium (II), (PdCl₂(dppf)); dichloro[1,4-bis(diphenylphosphino)butane]palladium (II) chloride, (PdCl₂(dppb)); dichlorobis(tricyclohexylphosphine)palladium (II), (PdCl₂(PCy₃)₂); dichloro[1,1'-bis(di-tert-butylphosphino)ferrocene]palladium (II), (PdCl₂(dtbp)); palladium; palladium chloride; palladium acetate; dichlorobis(triphenylphosphine)nickel (II), NiCl₂(PPh₃)₂; and mixtures of previously mentioned catalysts with phosphines. Preferably, the metal compound is tetrakis(triphenylphosphine)palladium (0), (Pd(PPh₃)₄) or NiCl₂(PPh₃)₂.

Preferably the coupling is carried out at a temperature comprising between room temperature and the reflux temperature of the solvent used. Preferably, the base used for the Suzuki coupling is selected from a metal alkaline carbonate such as sodium or potassium carbonate and a metal alkaline phosphate such as sodium or potassium phosphate. More preferably, the base is potassium phosphate.

The compounds of formula (II) are intermediates useful for the preparation of phenylnaphthalene-carboxylic acid derivatives of 1-adamantane, or a pharmaceutically acceptable salt thereof. In particular, the compounds of formula (IIa) are especially useful for the preparation of Adapalene.

Thus, the compounds of the present invention can be used in a preparation process of a compound of formula (I) or a pharmaceutically acceptable salt thereof, 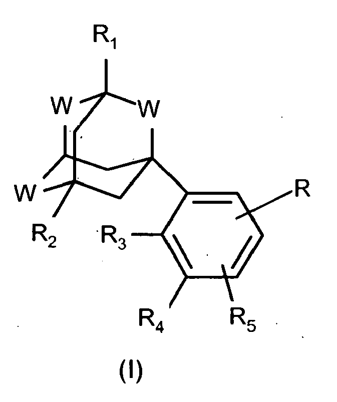 where: W is a biradical selected from the group consisting of: -CH₂-, -O-, and -SO₂-; R₁ and R₂ are radicals, equal or distinct, independently selected from the group consisting of H, halogen and a (C₁-C₆)-alkyl; R₃ is a radical selected from the group consisting of hydroxyl, acyl, amide, halogen; (C₁-C₆)-alkyl optionally substituted with one or more hydroxyl or acyl groups, and (C₁-C₄)-alkoxy optionally substituted for one or more hydroxyl, (C₁-C₄)-alkoxy or amide groups, and/or optionally interrupted by one or more oxygen atoms; R₄ is a radical selected from the group consisting of H, hydroxyl, (C₁-C₆)-alkyl, and (C₁-C₄)-alkoxy; or R₃ and R₄ form together a biradical -OCH₂O-; R₅ is a radical selected from the group consisting of H, (C₁-C₆)-alkyl, (C₁-C₄)-alkoxy, and a halogen; R is a radical selected from (R)-1 and (R)-2: 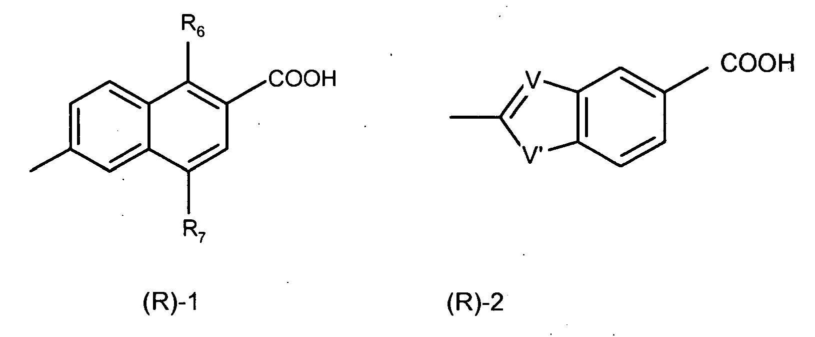 where: R₆ is a radical selected from H, (C₁-C₆)-alkyl, and halogen; R₇ is a radical selected from H, hydroxyl and halogen; V is a biradical -CH- and V' is an O atom; or V is an N atom and V' is a biradical -NH-; said process comprises submitting a compound of formula (II) as defined previously to a hydrolysis reaction.

In a preferred embodiment, the compound of formula (I) is the compound of formula (Ia) and the compound of formula (II) is the compound of formula (IIa).

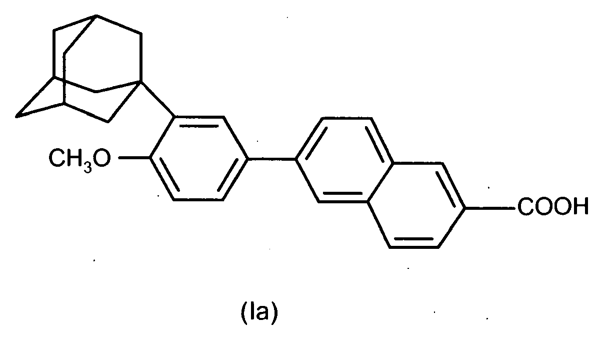

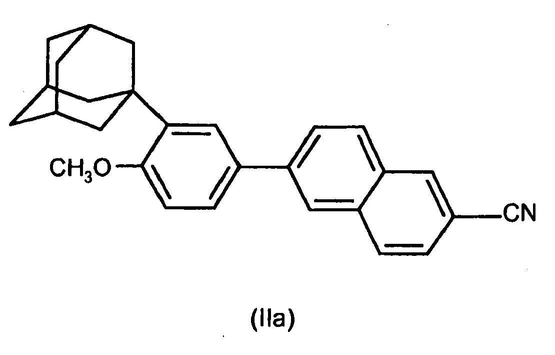

The most adequate conditions for carrying out the hydrolysis reaction vary depending on the parameters considered by one skilled in the art, such as the solvent used, the temperature, the time of hydrolysis, etc. These conditions can easily be determined by said expert in the art by routine tests, and with the help of the instructions from the examples given in this document. Preferably the hydrolysis is carried out with a base, optionally with a posterior acid treatment to isolate Adapalene. More preferably the base is selected from between a metal alkaline hydroxide such as potassium or sodium hydroxide.

Finally, the compound of formula (I) obtained can be converted into pharmaceutically acceptable salts thereof, or the pharmaceutically acceptable salts thereof can be converted into other salts by conventional methods.

An advantage of the present invention lies in the fact that this preparation process for aromatic derivatives of 1-adamantane provides a short, efficient and selective synthesis. In particular, the preparation of Adapalene by this process takes place with a high yield and is particularly advantageous in its practical industrial manufacture due to the fact that the use of naphthalene-carboxylic acid derivatives, which are expensive and hard to obtain, is avoided. In addition, the final product is obtained with a high chemical purity. An additional advantage of the process of the present invention lies in the fact that the protection/deprotection steps of the carboxyl group are not needed.

### EXAMPLES

Unless otherwise indicated, all of the reagents were used as they were received from the respective commercial suppliers. Tetrahydrofuran (THF) and dioxane were distilled over Na/benzophenone and toluene over Na. K₃PO₄ was finely ground before being used.

### Preparative Example 1: Preparation of 6-bromo-2-naphthalenyl trifluoromethanesulfonate

To a solution of 6-bromo-2-naphthol (2 g, 9.0 mmol) and triethylamine (NEt₃) (1.52 ml, 1.09 g, 10.8 mmol) in dichloromethane (CH₂Cl₂) (40 ml) and under an inert atmosphere at -10 °C, triflic anhydride [(CF₃SO₂)₂O, 1.8 ml, 3.03 g, 10.8 mmol] was added. After 2 h of stirring at -10 °C, the reaction mixture was diluted in H₂O (50 ml) and extractions were carried out with CH₂Cl₂ (3 x 40 ml). The combined organic phases were washed with HCl aq. (50 ml, 0.1 M), followed by H₂O (50 ml) and dried over Na₂SO₄. The filtration, evaporation and purification by column chromatography (SiO₂, CH₂Cl₂) led to the title compound (3.25 g) in the form of a colorless oil. IR (KBr) 3090, 1590, 1501, 1425, 1363, 1251, 1212, 1182, 1141, 1111, 1065, 960, 915, 882, 850, 801, 786, 767, 714, 653 and 609. M/Z (IQ, NH₃) 356 [M+ (⁸¹Br), 53%], 354 [M⁺ (⁷⁹Br), 63], 223 [M-SO₂CF₃⁺ (⁸¹Br), 63] and 221 [M-SO₂CF₃⁺ (⁷⁹Br), 100].

### Preparative Example 2: Preparation of 6-cyano-2-naphthalenyl trifluoromethanesulfonate

To a solution of 6-cyano-2-naphthol (1 g, 5.91 mmol) and anhydrous NEt₃ (anh.) (1 ml, 7.09 mmol) in CH₂Cl₂ anh. (20 ml) and under an inert atmosphere at 0 °C, triflic anhydride [(CF₃SO₂)₂O, 1.20 ml, 7.09 mmol] was added, dropwise. The reaction was brought to room temperature and was then left to react until there was no starting product observed (48 h). The reaction mixture was concentrated to dryness and the crude product obtained was suspended in EtOH (5 mL). Water (5 mL) was added, the mixture was triturated and the resulting suspension was filtered to give a crude product (1.74 g) as a brownish solid. Purification by column chromatography (SiO₂, CH₂Cl₂: cyclohexane, 6/4) yielded the compound of the title (1.56 g, 88%) in the form of a white solid IR (KBr) 3058, 2240, 1809, 1630, 1604, 1425, 1152, 964, 932. ¹H NMR (400 MHz, CDCl₃) 8.30 (d, J = 0.4 Hz, 1 H), 8.03 (d, J = 8.8 Hz, 1 H), 7.99 (d, J = 8.4 Hz, 1 H), 7.83 (d, J = 2.4 Hz, 1 H), 7.73 (dd, J = 8.8 and 1.4 Hz, 1 H) and 7.52 (dd, J = 8.8 and 2.4 Hz, 1 H).

### Preparative Example 3: Preparation of 6-cyano-2-naphthalenyl methanesulfonate

Et₃N (4.32 ml, 31.00 mmol) was added at 0°C and under inert atmosphere to a solution of 6-cyano-2-naphthol (4.74 g, 28.02 mmol) in anhydrous toluene (47 ml). The solution was stirred for 10 minutes at 0 °C. Next, MsCl (3.27 ml, 42.46 mmol) was added dropwise. The mixture was stirred at room temperature until no remaining reagent was observed (12 hours by thin-film chromatography). After washing with H₂O (3 x 25 ml), the organic phase was dried with MgSO₄ and was concentrated to dryness resulting in a crude in the form of an orange solid (7.52 g). Purification by column chromatography (SiO₂, CH₂Cl₂:cyclohexane, 8/2) resulted in the title compound (6.45 g, 93%) in the form of a beige solid. ¹H-NMR (400 MHz, CDCl₃) 8.27 (s, 1H), 7.71 (d, J = 8.8 Hz, 2H), 7.94 (d, J = 8.8 Hz, 1H), 7.83 (d, J = 2 Hz, 1H), 7.70 (dd, J = 8.8, 1.6 Hz, 1H), 7.54 (dd, J = 8.8, 2.4 Hz, 1H), 3.25 (s, 3H). M/Z (IQ, NH₃) 247 [M+, 14.78], 265 [M+18, 100]

### Preparative Example 4: Preparation of 6-cyano-2-naphthalenyl toluenesulfonate

Et₃N (1.10 ml, 7.85 mmol) was added, at 0°C and under inert atmosphere, to a solution of 6-cyano-2-naphthol (1.20 g, 7.09 mmol) in anhydrous CH₂Cl₂ (8 ml). The solution was stirred for 10 minutes at 0 °C. TsCl (1.52 g, 7.80 mmol) was added and the mixture was stirred at room temperature until no remaining reagent was observed (4 hours by thin-film chromatography). After washing with H₂O (3 x 10 ml), the organic phase was dried over MgSO₄ and was concentrated to dryness resulting in a crude in the form of a greyish solid (2.24 g). Purification by column chromatography (SiO₂, CH₂Cl₂:cyclohexane, 6/4) resulted in the title compound (2.07 g, 90%) in the form of a beige solid.
¹H-NMR (400 MHz, CDCl₃) 8.21 (s, 1H), 7.85 (d, J = 4 MHz, 1H), 7.83 (d, J = 4.4 MHz, 1H), 7.74 (d, J = 8.4 MHz, 1H), 7.63 (dd, J = 8.8, 1.6 MHz, 1H), 7.57 (d, J = 2.4 MHz, 1H), 7.33 (d, J = 8 MHz, 1H), 7.23 (dd, J = 8.8, 2.4 MHz, 1H), 2.46 (s, 3H). M/Z (IQ, NH₃) 323.0 [M+, 6.64], 341.1 [M+18, 100]

### Preparative Example 5: Preparation of 2,4,6-tris[3-(1-adamantyl)-4-methoxyphenyl]-1,3,5,2,4,6-trioxatriborinane

To a solution of 3-(1-adamantyl)-1-bromo-4-methoxybenzene (6 g, 18.7 mmol) in THF (90 ml) at -78 °C and under an inert atmosphere, n-BuLi (9 ml, 22.4 mmol, 2.5 M in hexane) was added during a period of 10 min. The reaction mixture was stirred at the same temperature for an hour, during which time a white precipitate formed. With the addition of B(O-i-Pr)₃ (15 ml, 65.4 mmol) at -78 °C the precipitate dissolved. After an hour of stirring at -78 °C, the reaction mixture was brought to room temperature and was stirred for 16 h. Next, the mixture was cooled to 0 °C and H₂O (6 ml) and HCl (6 ml, 2 M) were added. After 5 minutes, HCl (120 ml, 2 M) was again added and a vigorous stirring was maintained for 10 minutes. Finally, extractions were performed with EtOAc (3 x 100 ml). The combined organic phases were dried with Na₂SO₄, were filtered and after evaporation to dryness crude 3-(1-adamantyl)-4-methoxyphenylboronic acid (6.46 g, that contains some trimer) was obtained as a yellow solid.

The solid obtained was suspended in hexane (60 ml) and the suspension obtained was heated to 50 °C for 30 min. Next, the suspension was left to cool to room temperature, it was filtered and the solid was washed with hexane (30 ml). Once dried in vacuum, the title compound was obtained (5.53 g) as a white solid that was used in subsequent Suzuki couplings without prior purification. IR (KBr) 3228, 2902, 2846, 1597, 1453, 1400, 1339, 1281, 1235, 1181, 1138, 1100, 1022, 820, 758 and 724. ¹H NMR (400 MHz, CDCl₃) 8.15 (s, 1 H), 8.05 (d, J = 8.4 Hz, 1 H), 7.00 (d, J = 8.4 Hz, 1 H), 3.92 (s, 3 H), 2.21 (s, 6 H), 2.10 (s, 3 H) and 1.82 (s, 6 H).

### Preparative Example 6: Preparation of (3-(1-adamantyl)-4-methoxyphenyl)-5,5-dimethyl-1,3,2-dioxaborinane

A solution of 2,4,6-tris[3-(1-adamantyl)-4-methoxyphenyl]-1,3,5,2,4,6-trioxatriborinane (1 g, 1.24 mmol) and 2,2-dimethyl-propan-1,3-diol (388 mg, 3.73 mmol) in toluene (10 ml) provided with a Dean-Stark collector was heated to reflux under an inert atmosphere for 6 h. The toluene was evaporated at reduced pressure and cyclohexane (2 ml) added. After heating the solution to reflux during 10 min, this was cooled to room temperature and after filtering the title compound (904 mg, 68%) was obtained as a white solid, which was used in subsequent Suzuki couplings without previous purification. IR (KBr) 3217, 2958, 2900, 1596, 1477, 1416, 1377, 1310, 1283, 1233, 1177, 1136, 1100, 1032, 990, 816, 694, 676 and 633. M/Z (Electrospray) 355 (M⁺).

### Preparative Example 7: Preparation of 6-[3-(1-adamantyl)-4-methoxyphenyl]-2-bromonaphthalene

3-(1-Adamantyl)-4-methoxyphenyl)boronic acid (150 mg, 0.32 mmol), 6-bromo-2-naphthalenyl trifluoromethanesulfonate (93 mg, 0.26 mmol), K₃PO₄ (222 mg, 1.05 mmol), KBr (34 mg, 0.29 mmol) and THF (2 ml) were placed in a Schlenk tube. The reaction mixture was deoxygenated (3 freeze-thaw cycles). Immediately after, Pd(PPh₃)₄ (15 mg, 0.013 mmol) was added and the mixture was again deoxygenated (2 freeze-thaw cycles). After heating to reflux for 18 h, the mixture was brought to room temperature and was diluted with CHCl₃ (5 ml). The solution was filtered through celite and washings with CHCl₃ (2 x 5 ml) were performed. Evaporation of the combined organic phases yielded a residue that was redissolved in CHCl₃ (5 ml) and washed with H₂O (2 x 5 ml). The organic phase was dried over Na₂SO₄ and after evaporating to dryness, a crude product (97 mg) was obtained that was recrystallized with a minimum volume of toluene at reflux. The title compound (68 mg, 58%) was obtained as a pale yellow powder. IR (KBr) 2900, 2847, 1600, 1489, 1456, 1442, 1262, 1237, 1178, 1142, 1103, 1061, 1026, 877, 809 and 470. M/Z (EI) 448 [M⁺ (⁸¹Br), 76%] and 446 [M⁺ (⁷⁹Br), 100].

### Preparative Examples 8: Preparation of 6-[3-(1-adamantyl)-4-methoxyphenyl]-2-bromonaphthalene

2,4,6-Tris[3-(1-adamantyl)-4-methoxyphenyl]-1,3,5,2,4,6-trioxatriborinane (151 mg, 0.19 mmol), 6-bromo-2-naphthalenyl trifluoromethanesulfonate (100 mg, 0.28 mmol), K₃PO₄ (239 mg, 1.13 mmol), THF (2 ml) and H₂O (0.4 ml) were placed in a Schlenk tube. The mixture was deoxygenated (3 freeze-thaw cycles). Immediately after, Pd(PPh₃)₄ (16 mg, 0.014 mmol) was added and the reaction mixture was again deoxygenated (2 freeze-thaw cycles). After heating to reflux for 15 h, the mixture was diluted, still hot, in toluene (5 ml). The solution was filtered through celite which was washed with hot toluene (2 x 5 ml). The combined organic phases were washed with hot H₂O (2 x 5 ml). The organic phase was evaporated to dryness and yielded a crude product (201 mg) that was recrystallized with the minimum volume of toluene (1.2 ml) at reflux, obtaining the title compound (107 mg, 87%) as a pale yellow powder. The spectroscopic data coincide with those of Example 7.

### Preparative Example 9: Preparation of 6-[3-(1-adamantyl)-4-methoxyphenyl]-2-bromonaphthalene

(3-(1-Adamantyl)-4-methoxyphenyl)-5,5-dimethyl-1,3,2-dioxaborinane (100 mg, 0.28 mmol), 6-bromo-2-naphthalenyl trifluoromethanesulfonate (67 mg, 0.19 mmol), K₃PO₄ (160 mg, 0.75 mmol), THF (2 ml) and H₂O (0.4 ml) were placed in a Schlenk tube. The reaction mixture was deoxygenated (3 freeze-thaw cycles). Immediately after, Pd(PPh₃)₄ (11 mg, 0.009 mmol) was added and the mixture was again deoxygenated (2 freeze-thaw cycles). After heating to reflux for 17 h, the mixture was diluted, still hot, with toluene (5 ml). The solution was filtered through celite which was washed with hot toluene (2 x 5 ml). The organic phase was evaporated and a crude product (59 mg) was obtained that was recrystallized with a minimum volume of toluene at reflux (0.55 ml) giving the title compound (20 mg, 24%) as a pale yellow powder. The spectroscopic data coincide with those of Example 7.

### Preparative Example 10: Preparation of 6-[3-(1-adamantyl)-4-methoxyphenyl]-2-bromonaphthalene

2,4,6-Tris[3-(1-adamantyl)-4-methoxyphenyl]-1,3,5,2,4,6-trioxatriborinane (187 mg, 0.23 mmol), 2,6-dibromonaphthalene (100 mg, 0.35 mmol), K₃PO₄ (296 mg, 1.4 mmol), THF (2 ml) and H₂O (0.4 ml) were placed in a Schlenk tube. The reaction mixture was deoxygenated (3 freeze-thaw cycles). Immediately after, Pd(PPh₃)₄ (20 mg, 0.017 mmol) was added and the mixture was again deoxygenated (2 freeze-thaw cycles). After heating to reflux during 15 h, the reaction mixture was diluted, still hot, in toluene (5 ml). The mixture was filtered through celite which was washed with hot toluene (2 x 5 ml). The combined organic phases were washed with hot H₂O (2 x 5 ml). The organic phase was evaporated and a crude product (128 mg) was obtained which was recrystallized with a minimum volume of toluene at reflux (0.75 ml), obtaining the title compound (39 mg, 25%) as a pale yellow powder. The spectroscopic data coincide with those of Example 7.

### Example 11: Preparation of 6-[3-(1-adamantyl)-4-methoxyphenyl]-2-cyanonaphthalene

To a suspension of 6-[3-(1-adamantyl)-4-methoxyphenyl]-2-bromonaphthalene (250 mg, 0.56 mmol) in DMF (2 mL), CuCN (90 mg, 1.01 mmol) was added and the mixture was stirred at 160 °C for 8 h. The reaction was cooled to 100 °C and was added to a solution of NH₃ aq. (5 ml, 14%). The resulting mixture was extracted with toluene (3 x 10 ml) and the combined organic phases were washed with NH₃ aq (3 x 10 ml, 5%) and evaporated to dryness once dried with Na₂SO₄, leading to the crude intermediate cyanide (220 mg). Recrystallization in toluene at reflux yielded the desired product (200 mg, 91 %) as a brown solid. IR (KBr) 3398, 2898, 2840, 2219, 1604, 1476, 1239, 1027, 880 and 816. ¹H NMR (400 MHz, CDCl₃) 8.23 (s, 1 H), 8.01 (s, 1 H), 7.94 (t, J = 8.0 Hz, 2 H), 7.85 (dd, J = 8.4 and 1.6 Hz, 1 H), 7.60 (m, 2 H), 7.54 (dd, J = 8.4 y 2.4 Hz, 1 H), 7.00 (d, J = 8.4 Hz, 1 H), 3.91 (s, 3 H), 2.18 (s, 6 H), 2.11 (s, 3 H), 1.81 (s, 6 H). M/Z (EI) 393 (M+, 100).

### Example 12: Preparation of 6-[3-(1-adamantyl)-4-methoxyphenyl]-2-cyanonaphthalene

2,4,6-Tris[3-(1-adamantyl)-4-methoxyphenyl]-1,3,5,2,4,6-trioxatriborinane (889 mg, 1.11 mmol), 6-cyanonaphthalenyl trifluoromethanesulfonate (500 mg, 1.66 mmol), K₃PO₄ (1,41 g, 6.64 mmol), THF (10 ml) and H₂O (2 ml) were placed in a Schlenk flask and the reaction mixture was deoxygenated (3 freeze-thaw cycles). Immediately after, Pd(PPh₃)₄ (96 mg, 0.083 mmol) was added and the mixture was deoxygenated again (2 freeze-thaw cycles). After heating to reflux for 17 h, the reaction mixture was diluted, while still hot, in toluene (50 ml). The mixture was filtered through celite which was washed with hot toluene (2 x 50 ml). The combined organic phases were washed with hot H₂O (2 x 50 ml). The organic phase was evaporated and a crude product (906 mg) was obtained that was recrystallized with a minimum volume of toluene at reflux (6.2 ml), leading to the title compound (541 mg, 83%) as a gray powder. The spectroscopic data coincide with those of Example 11.

### Example 13: Preparation of 6-[3-(1-adamantyl)-4-methoxyphenyl]-2-cyanonaphthalene

2,4,6-Tris[3-(1-adamantyl)-4-methoxyphenyl]-1,3,5,2,4,6-trioxaborinane (69 mg, 0.086 mmol), 6-cyano-2-naphthalenyl methanesulfonate (50 mg, 0.202 mmol), NiCl₂(PPh₃)₂ (13 mg, 0.020 mmol), PPh₃ (11 mg, 0.040 mmol) and K₃PO₄·1.5 H₂O (290 mg, 1.213 mmol) were added to a Schlenk flask and the reaction mixture was deoxygenated (3 freeze/unfreeze cycles). Anhydrous toluene was added (1 ml). After heating the reaction mixture to 120 °C for 16 h, the reaction mixture was brought to room temperature and CH₂Cl₂ was added (3 ml). The suspension was filtered through celite and the solid was washed with CH₂Cl₂. The filtrate was dried over MgSO₄ and was concentrated to dryness resulting in a crude in the form of a yellow solid (108 mg). Purification by column chromatography (SiO₂, CH₂Cl₂: cyclohexane, 60/40) resulted in the title compound (75 mg, 94%). Spectroscopic data coincide with those of Example 11.

This coupling was suitably carried out also with xylene (58%), dioxane (65%) and THF (52%).

### Example 14: Preparation of 6-[3-(1-adamantyl)-4-methoxyphenyl]-2-cyanonaphthalene

To a mixture of 3-(1-adamantyl)-1-bromo-4-methoxybenzene (62 mg, 0.19 mmol), and THF anh. (1 ml) under Ar atmosphere and at -78 °C, t-BuLi (197 µl, 1.4 M, 0.27 mmol) was added, dropwise, and the mixture was stirred for 1 hour. After this time, the reaction was allowed to warm to room temperature, a solution of ZnCl₂ (26 mg, 0.19 mmol) in anhydrous THF (0.4 mL) was added and stirring was continued for another hour. A solution of 6-cyanonaphthalenyl trifluoromethanesulfonate (50 mg, 0.17 mmol), Pd(PPh₃)₄ (12 mg, 0.01 mmol) and anhydrous THF (0.2 ml) was added and the reaction was maintained for 18 h at room temperature and for 5 h at 50 °C. Finally, the reaction was neutralized with HCl (1M) and extractions were performed with Et₂O (3 x 2 ml). The organic phase was washed with NaCl sat (3 x 3 ml) and H₂O (3 x 3 ml), was dried with MgSO₄ and evaporated to dryness. A crude product was obtained (88 mg) in the form of an orange oil, which after purification by column chromatography (SiO₂, CH₂Cl₂:cyctohexane 8/2) yielded the title compound (11 mg, 18%) as a white solid. The spectroscopic data coincide with those of Example 11.

### Example 15: Preparation of 6-[3-(1-adamantyl)-4-methoxyphenyl]-2-cyanonaphthalene

A previously prepared solution of 3-(1-adamantyl)-1-bromo-4-methoxybenzene (50 mg, 0.156 mmol), dibromoethane (13 µl, 0.155 mmol) and anhydrous THF (0.4 ml) was added over a suspension of Mg (56.4 mg, 2.322 mmol) in anhydrous THF (1 ml), and the mixture was stirred at room temperature until the reaction was activated (2.5 min). Once the reaction was activated, a solution of 3-(1-adamantyl)-1-bromo-4-methoxybenzene (0.45 g, 1.402 mmol) in anhydrous THF (3 ml) was added dropwise and the reaction mixture was stirred at reflux temperature for 2 hours resulting in the organic magnesium compound. Next, the solution was cooled to room temperature and a fraction of said organic magnesium compound solution (0.858 ml, 0.304 mmol, 0.354 M) was added over ZnCl₂ (21.5 mg, 0.304 mmol) (previously melted under vacuum and cooled to room temperature under inert atmosphere) and the mixture was stirred at room temperature for 1 h resulting in the organic zinc derivative.

The previously prepared solution of the organic zinc derivative was added over a previously deoxygenated (3 freeze/unfreeze cycles) mixture of 6-cyano-2-naphthalenyl methanesulfonate (50 mg, 0.202 mmol), NiCl₂(PPh₃)₂ (13 mg, 0.020 mmol) and PPh₃ (11 mg, 0.040 mmol), and the mixture was stirred at room temperature for 16h.

Next, the solution was diluted with H₂O (2 ml) and extractions were carried out with CH₂Cl₂ (3 x 5 ml). The organic phase was washed with NaCl sat (3 x 3 ml), the organic phase was dried over MgSO₄ and was concentrated dryness resulting in a yellowish crude (115 mg). The purification by column chromatography (SiO₂, CH₂Cl₂:cyclohexane, 40/60) resulted in the title compound (47 mg, 59%) in the form of a white solid. Spectroscopic data coincide with those of Example 11.

### Example 16: Preparation of 6-[3-(1-adamantyl)-4-methoxyphenyl]-2-cyanonaphthalene

2,4,6-Tris[3-(1-adamantyl)-4-methoxyphenyl]-1,3,5,2,4,6-trioxaborinane (53 mg, 0.066 mmol), 6-cyano-2-naphthalenyl toluenesulfonate (50 mg, 0.155 mmol), NiCl₂(PPh₃)₂ (10 mg, 0.016 mmol), PPh₃ (8 mg, 0.031 mmol) and K₃PO₄·1.5 H₂O (222 mg, 0.930 mmol) were added to a Schlenk flask and the reaction mixture was deoxygenated (3 freeze/unfreeze cycles). Anhydrous toluene was added (2 ml). After heating to 85 °C for 16 h, the reaction mixture was cooled to room temperature and CH₂Cl₂ was added (3 ml). The suspension was filtered through celite and the solid was washed with CH₂Cl₂. The filtrate was dried with MgSO₄ and was concentrated to dryness resulting in a crude in the form of a yellow solid (128 mg). Purification by column chromatography (SiO₂, CH₂Cl₂:cyclohexane, 60/40) resulted in the title compound (33 mg, 54%). Spectroscopic data coincide with those of Example 11.

The reaction was also carried out in anhydrous toluene yielding the title compound in 41 % yield.

### Example 17: Preparation of 6-[3-(1-adamantyl)-4-methoxyphenyl]-2-cyanonaphthalene

3.5 ml of a previously prepared solution of 3-(1-adamantyl)-1-bromo-4-methoxybenzene (2 g, 6.23 mmol) in anhydrous THF (7 ml) was added to a suspension of Mg (166 mg, 6.83 mmol) in anhydrous THF (4 ml), and the mixture was stirred at room temperature for 10 min and then at 67°C until the reaction was activated. Once the reaction was activated, the remaining solution of 3-(1-adamantyl)-1-bromo-4-methoxybenzene in anhydrous THF was added dropwise, and the reaction mixture was stirred at 67°C for 1 hour resulting in the organic magnesium derivative. The solution was cooled to room temperature.

The previously prepared organic magnesium derivative (0.748 ml, 0.62 M, 0.464 mmol) was added to a mixture of cyanonaphthalenyl toluenesulfonate (50 mg, 0.155 mmol), NiCl₂(PPh₃)₂ (5 mg, 0.007 mmol) and PPh₃ (2 mg, 0.007 mmol) under inert atmosphere, and the solution was stirred at 80°C for 72 h.

The solution was cooled to room temperature, diluted in CH₂Cl₂ (3 ml), filtered and washed with CH₂Cl₂. The combined filtrates were dried over MgSO₄ and were concentrated to dryness resulting in a crude in the form of an orange oil (170 mg). Purification by column chromatography (SiO₂, CH₂Cl₂:cyclohexane, 60/40) resulted in the title compound (7 mg, 11 %). Spectroscopic data coincide with those of Example 11.

### Example 18: Preparation of 6-[3-(1-adamantyl)-4-methoxyphenyl]-2-hydroxynaphthalene

2,4,6-Tris[3-(1-adamantyl)-4-methoxyphenyl]-1,3,5,2,4,6-trioxatriborinane (1.18 g, 1.46 mmol), 6-bromo-2-naphtol (500 mg, 2.19 mmol), Na₂CO₃ (0.93 g, 8.77 mmol), toluene (4 ml), ethanol (4 ml) and H₂O (4 ml) were placed in a Schlenk flask and the reaction mixture was deoxygenated (3 freeze-thaw cycles). Immediately after, Pd(PPh₃)₄ (127 mg, 0.11 mmol) was added and the mixture was deoxygenated again (2 freeze-thaw cycles). After heating at reflux temperature for 17 h, the reaction mixture was diluted, while still hot, in toluene (60 ml). The mixture was filtered through celite which was washed with hot toluene (2 x 50 ml). The combined organic phases were washed with hot H₂O (2 x 50 ml). The organic phase was evaporated and a crude product was obtained (1.24 g). Purification with column chromatography (SiO₂, CH₂Cl₂: cyclohexane, 8/2) gave the title compound (0.46 g, 55%) in the form of a white solid. IR (KBr) 3515, 3361, 2900, 2848, 1604, 1497, 1236, 1181, 1139, 1026, 860, 805; ¹H NMR (400 MHz, CDCl₃) 8.01 (s, 1 H), 7.95 (d, J = 9.2 Hz, 1 H), 7.90 (d, J = 8.4 Hz, 1 H), 7.83 (dd, J = 8.4 y 2 Hz, 1 H), 7.75 (d, J = 2.4, 1 H), 7.57 (d, J = 2.4 Hz, 1 H), 7.52 (dd, J = 8.4 and 2.4 Hz, 1 H), 7.37 (dd, J = 9.2 and 2.4, 1H), 7.00 (d, J = 8.4 Hz, 1 H), 3.90 (s, 3 H), 2.19 (s, 6 H), 2.10 (s, 3 H) and 1.80 (s, 6 H). M/Z (EI) 516 ( M+, 100 %)

### Example 19: Preparation of 6-[3-(1-adamantyl)-4-methoxyphenyl]-2-naphthalenyl trifluoromethanesulfonate

To a solution of 6-[3-(1-adamantyl)-4-methoxyphenyl]-2-hydroxynaphthalene (0.46 g, 1.20 mmol) and NEt₃ anh. (0.20 ml, 1.44 mmol) in CH₂Cl₂ anh. (9.2 ml) under inert atmosphere at 0 °C, triflic anhydride [(CF₃SO₂)₂O, 0.24 ml, 1.44 mmol] was added dropwise. The reaction was brought to room temperature was left to react until no more starting product was observed (18 h). The reaction mixture was concentrated to dryness and the crude product obtained was suspended in EtOH (5 ml). Water (8 ml) was added, the mixture was triturated and the resulting suspension was filtered to give a crude (0.514 g) as a grey-brown solid. Purification by column chromatography (SiO₂, CH₂Cl₂ 100%) yielded the title compound (0.454 g, 73%) in the form of a yellowish solid. IR (KBr) 3449, 2904, 2847, 1610, 1502, 1412, 1143, 1027, 931, 810, 637. ¹H NMR (400 MHz, CDCl₃) 7.91 (s, 1 H), 7.79 (d, J = 8.8 Hz, 1 H), 7.72 (d, J = 8.4 Hz, 1 H), 7.68 (dd, J = 8.4 and 2.4 Hz, 1 H), 7.56 (d, J = 2.4 Hz, 1 H), 7.49 (dd, J = 8.4 and 2.4 Hz, 1 H), 7.16 (d, J = 2.4 Hz, 1 H), 7.12 (dd, J = 8.8, 2.4 Hz, 1 H), 6.98 (d, J = 8.4 Hz, 1 H), 4.93 (sa, OH), 3.89 (s, 3H), 2:18 (s, 6H), 2.10 (s, 3H) and 1.80 (s, 6H). M/Z (IQ) 534 (M + NH₃, 33), 516 (M+, 100), 383 (84)

### Example 20: Preparation of 6-[3-(1-adamantyl)-4-methoxyphenyl]-2-cyanonaphthalene

To a mixture of 6-[3-(1-adamantyl)-4-methoxyphenyl]-2-naphthalenyl trifluoromethanesulfonate (60 mg, 0.12 mmol) in DMF anh (1.1 ml), was added Zn(CN)₂ (8.2 mg, 0.07 mmol) and Pd(PPh₃)₄ (6 mg, 0.005 mmol) and the reaction mixture was stirred at 80 °C for 1 hr. The reaction was diluted in Et₂O (3 mL) and washings were carried out with H₂O (3 x 4 ml). The organic phase was dried with MgSO₄ and evaporated to dryness leading to the crude product (40 mg) as a white solid. Purification by column chromatography (SiO₂, CH₂Cl_{2:} cyclohexane, 6/4) yielded the title compound (24 mg, 53%) in the form of a white solid. The spectroscopic data coincide with those of Example 11.

### Preparative Example 21: Preparation of Adapalene

To a mixture of 6-[3-(1-adamantyl)-4-methoxyphenyl]-2-cyanonaphthalene (125 mg, 0.32 mmol), dioxane (2 mL), MeOH (2 mL) and H₂O (0.8 mL), was added KOH (222 mg, 3.97 mmol) and the reaction mixture was stirred at reflux temperature for 24 h. The mixture was acidified with HCl (5 mL, 2M) was extracted with EtOAc (3 x 10 mL). The combined organic phases were evaporated to dryness once dried with Na₂SO₄, giving crude Adapalene (164 mg). Recrystallization from toluene yielded Adapalene (122 mg, 58%) as a white solid.

## Claims

1. A compound of formula (II), 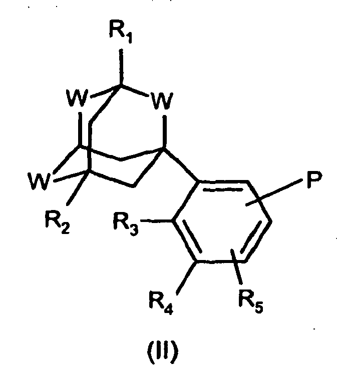 wherein:
W is a biradical selected from the group consisting of: -CH₂, -O-, and -SO₂-;
R₁ and R₂ are radicals, equal or distinct, independently selected from the group consisting of H, halogen and (C₁-C₆)-alkyl;
R₃ is a radical selected from the group consisting of hydroxyl, acyl, amide, halogen, alkyl (C₁-C₆) optionally substituted by one or more hydroxyl or acyl groups, and (C₁-C₄)-alkoxy optionally substituted by one or more hydroxyl, (C₁-C₄)-alkoxy or amino carbonyl groups, and/or optionally interrupted by one or more atoms of oxygen;
R₄ is a radical selected from the group consisting of H, hydroxyl, (C₁-C₆)-alkyl, and (C₁-C₄)-alkoxy;
or R₃ and R₄ form together a biradical -OCH₂O-;
R₅ is a radical selected from the group consisting of H, (C₁-C₆)-alkyl, (C₁-C₄)-alkoxy, and a halogen; and
P is a radical selected from (P)-1 and (P)-2: 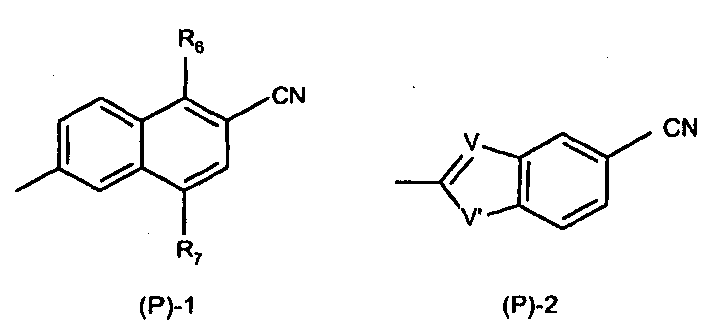
wherein:
R₆ is a radical selected from H, (C₁-C₆)-alkyl, and halogen;
R₇ is a radical selected from H, hydroxyl and halogen; and
V is a biradical -CH- and V' is an O atom; or V is an N atom and V' is a biradical -NH-.

2. The compound according to claim 1, wherein W is -CH₂-.

3. The compound according to claim 2, wherein P is a radical (P)-1.

4. The compound according to claim 3, wherein the compound (II) is the compound of formula (IIa).

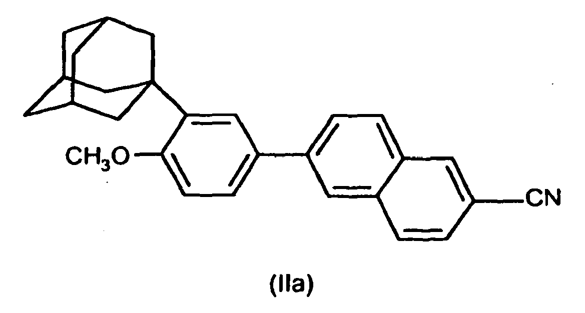

5. A preparation process of a compound of formula (II) as defined in any of the claims 1-4, which comprises reacting a compound of formula (III), 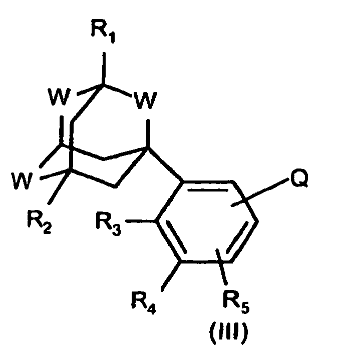 where Q is a radical selected from the following formulae: 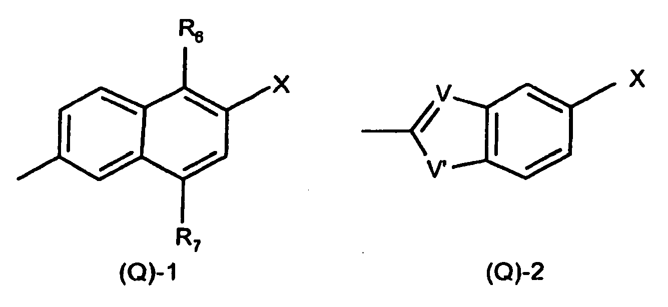 where X is a leaving group selected from a halogen that is selected from Cl, Br and I, and a sulfonate of formula -OSO₂R₉ where R₉ is selected from the group consisting of CF₃, (C₁-C₄)-alkyl, phenyl, and phenyl mono- or disubstituted by a radical selected from (C₁-C₄)-alkyl, halogen and nitro; and W, V, V', R₁, R₂, R₃, R₄, R₅, R₆ and R₇ have the same meaning as defined above for the compound of formula (II),
with a metal cyanide.

6. The process according to claim 5, where the compound of formula (III) is the compound of formula (IIIa) where X has the same meaning as in claim 5.

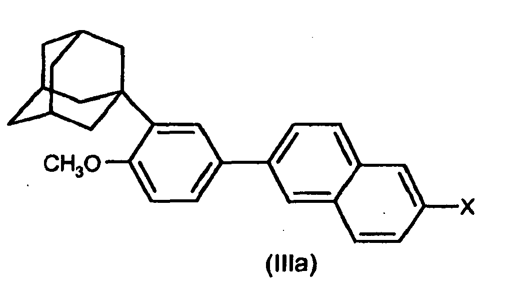

7. The process according to claim 6, wherein the halogen is Br.

8. The process according to claim 7, wherein the reaction is carried out with copper cyanide.

9. The process according to claim 6, wherein R₉ is CF₃.

10. The process according to claim 9, wherein the reaction is carried out with zinc cyanide.

11. The process according to any of the claims 9-10, wherein the reaction is carried out in the presence of a palladium or nickel catalyst.

12. The process according to claim 11, wherein the catalyst is a palladium catalyst.

13. A compound of formula (X), wherein R₉ is a radical that is selected from CF₃, (C₁-C₄)-alkyl, phenyl, and phenyl that is disubstituted by a (C₁-C₄)-alkyl radical.

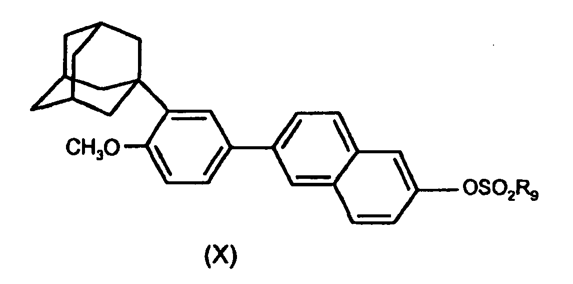

14. The compound according to claim 13, wherein R₉ is -CF₃.

15. A compound of formula (IX).

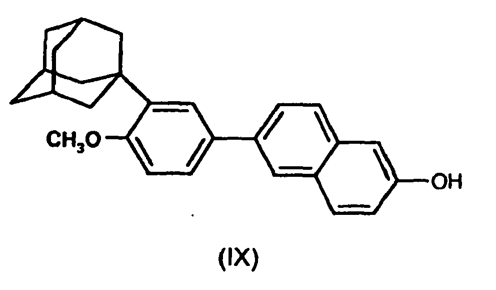

## Patentansprüche

1. Verbindung der Formel (II), 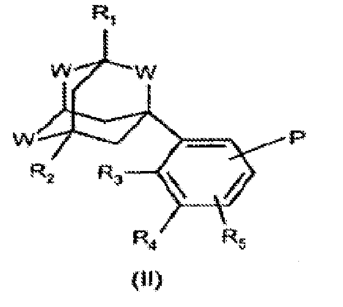 wobei:
• W ein Diradikal ist ausgewählt aus der Gruppe bestehend aus: -CH₂-, -0- und -SO₂-;
• R₁ und R₂ gleiche oder verschiedenartige Radikale sind, die unabhängig aus der Gruppe ausgewählt sind bestehend aus H, Halogen und (C₁-C₆)-Alkyl;
• R₃ ein Radikal ist ausgewählt aus der Gruppe bestehend aus Hydroxyl, Acyl, Amid, Halogen, Alkyl (C₁-C₆), das wahlweise durch eine oder mehrere Hydroxyl- oder Acylgruppen substituiert ist, und (C₁-C₄)-Alkoxy, das wahlweise durch ein oder mehrere Hydroxyl-, (C₁-C₄)-Alkoxy- oder Aminocarbonylgruppem substituiert und/oder wahlweise durch ein oder mehrere Sauerstoffatome unterbrochen ist;
• R₄ ein Radikal ist ausgewählt aus der Gruppe bestehend aus H, Hydroxyl, (C₁-C₆)-Alkyl und (C₁-C₄)-Alkoxy; oder R₃ und R₄ zusammen ein Diradikal -OCH₂O- bilden;
• R₅ ein Radikal ist ausgewählt aus der Gruppe bestehend aus H, (C₁-C₆)-Alkyl, (C₁-C₄)-Alkoxy und einem Halogen; und
• P ein Radikal ist ausgewählt von (P)-1 und (P)-2: 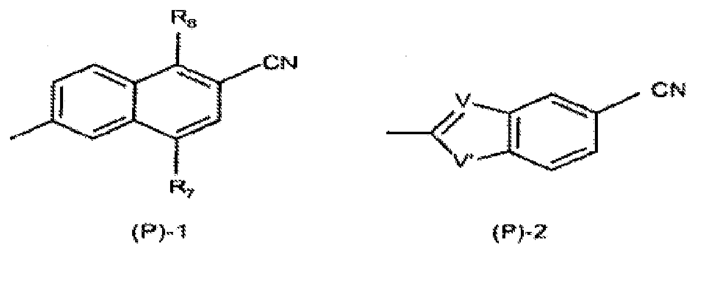 wobei:
• R₆ ein Radikal ist ausgewählt unter H, (C₁-C₆)-Alkyl und Halogen; und
• V ein Diradikal -CH- ist und V' ein O-Atom ist; oder V ein N-Atom ist und V' ein Diradikal -NH- ist.

2. Verbindung nach Anspruch 1, wobei W -CH₂- ist.

3. Verbindung nach Anspruch 2, wobei P ein Radikal (P)-1 ist.

4. Verbindung nach Anspruch 3, wobei die Verbindung (II) die Verbindung der Formel (IIa) 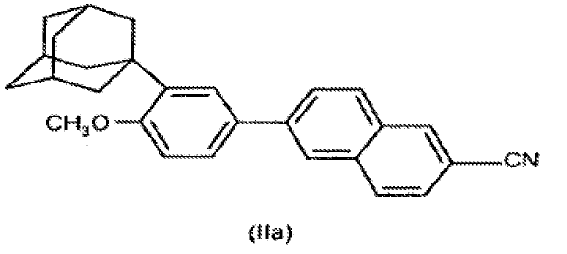 ist.

5. Herstellungsverfahren für eine Verbindung der Formel (II) wie in einem der Ansprüche 1 - 4 definiert, das das Reagieren einer Verbindung der Formel (III), 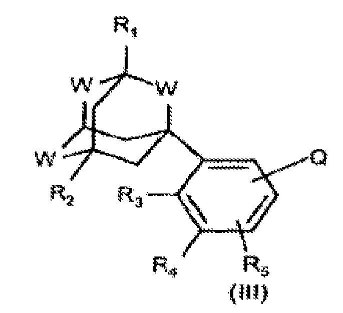 wobei Q ein Radikal ist ausgewählt aus den folgenden Formeln: 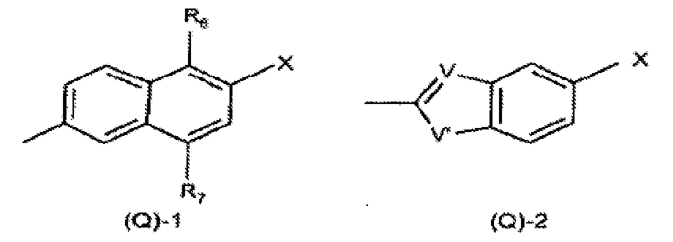 wobei X eine Austrittsgruppe ist ausgewählt unter einem Halogen, das aus Cl, Br und I ausgewählt ist, und einem Sulfonat der Formel -OSO₂R₉, wobei R₉ aus der Gruppe ausgewählt ist bestehend aus CF₃, (C₁-C₄)-Alkyl, Phenyl und Phenyl, das durch ein Radikal mono- oder disubstituiert ist ausgewählt unter (C₁-C₄)-Alkyl, Halogen und Nitro; und W, V, V', R₁, R₂, R₃, R₄, R₅, R₆ und R₇ dieselbe Bedeutung besitzen wie oben für die Verbindung der Formel (II) definiert, mit einem Metallcyanid umfasst.

6. Verfahren nach Anspruch 5, wobei die Verbindung der Formel (III) die Verbindung der Formel (IIIa) ist, wobei X dieselbe Bedeutung besitzt wie in Anspruch 5.

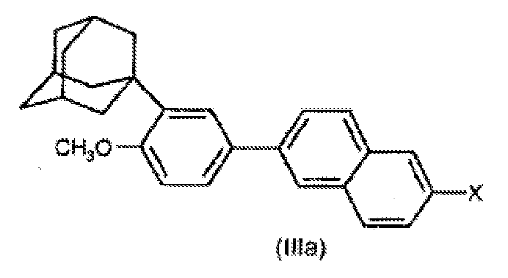

7. Verfahren nach Anspruch 6, wobei das Halogen Br ist.

8. Verfahren nach Anspruch 7, wobei die Reaktion mit Kupfercyanid durchgeführt wird.

9. Verfahren nach Anspruch 6, wobei R₉ CF₃ ist.

10. Verfahren nach Anspruch 9, wobei die Reaktion mit Zinkcyanid durchgeführt wird.

11. Verfahren nach einem der Ansprüche 9 - 10, wobei die Reaktion in Gegenwart eines Palladium- oder Nickelkatalysators durchgeführt wird.

12. Verfahren nach Anspruch 11, wobei der Katalysator ein Palladiumkatalysator ist.

13. Verbindung der Formel (X), wobei R₉ ein Radikal ist, das unter CF₃, (C₁-C₄)-Alkyl, Phenyl und Phenyl, das durch ein (C₁-C₄)-Radikal disubstituiert ist, ausgewählt ist.

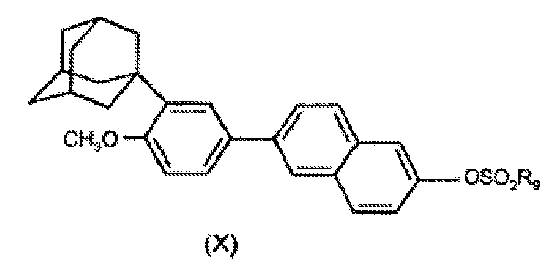

14. Verbindung nach Anspruch 13, wobei R₉ -CF₃ ist.

15. Verbindung der Formel (IX).

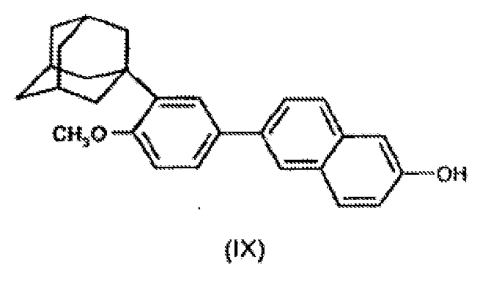

## Revendications

1. Un composé de formule (II), 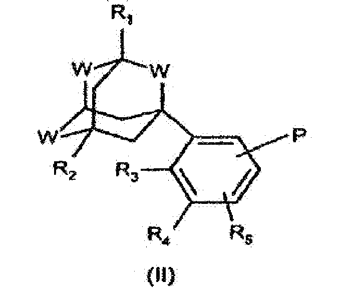 dans laquelle:
• W est un biradical choisi dans le groupe consistant en: -CH₂-, -O- et -SO₂-;
• R₁ et R₂ sont des radicaux, égaux ou différents, indépendamment choisis dans le groupe consistant en l'atome d'hydrogène, un atome d'halogène et un groupe alkyle (en C₁ à C₆);
• R₃ est un radical choisi dans le groupe consistant en un groupe hydroxyle, acyle, amide, un atome d'halogène, un groupe alkyle (en C₁ à C₆) facultativement substitué par un ou plusieurs groupes hydroxyle ou acyle; et alcoxy (en C₁ à C₄) facultativement substitué par un ou plusieurs groupes hydroxyle, alcoxy (en C₁ à C₄) ou aminocarbonyle, et/ou facultativement interrompu par un ou plusieurs atomes d'oxygène;
• R₄ est un radical choisi dans le groupe consistant en l'atome d'hydrogène, un groupe hydroxyle, alkyle (en C₁ à C₆) et alcoxy (en C₁ à C₄);
ou bien R₃ et R₄ forment ensemble un biradical -OCH₂O-;
• R₅ est un radical choisi dans le groupe consistant en l'atome d'hydrogène, un groupe alkyle (en C₁ à C₆), alcoxy (en C₁ à C₄), et un atome d'halogène; et
• P est un radical choisi parmi (P)-1 et (P)-2: 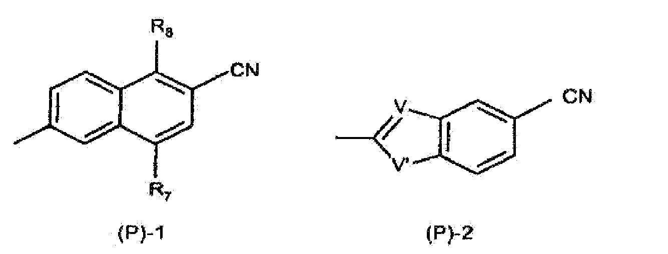
dans lesquels:
• R⁶ est un radical choisi parmi l'atome d'hydrogène, un groupe alkyle (en C₁ à C₆) et un atome d'halogène;
• R⁷ est un radical choisi parmi en l'atome d'hydrogène, un groupe hydroxyle et un atome d'halogène; et
• V est un biradical -CH- et V' est un atome O; ou V est un atome N et V' est un biradical -NH-.

2. Le composé selon la revendication 1, dans lequel W est -CH₂-.

3. Le composé selon la revendication 2, dans lequel P est un radical (P)-1.

4. Le composé selon la revendication 3, dans lequel le composé (II) répond à la formule (IIa):

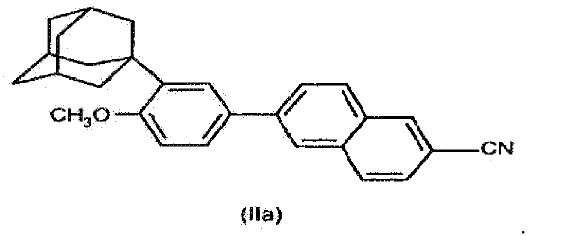

5. Le procédé de préparation d'un composé de formule (II) tel que défini dans l'une quelconque des revendications 1 à 4, qui comprend la réaction avec un cyanure de métal d'un composé de formule (III), 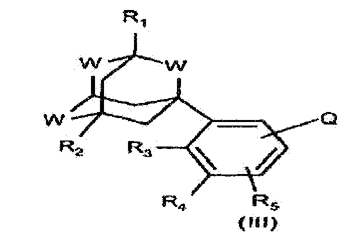 où Q est un radical choisi parmi les formules suivantes: 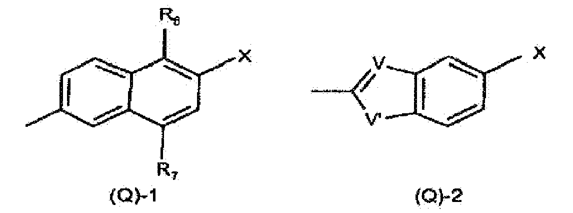 où X est un groupe partant choisi parmi un atome d'halogène choisi parmi Cl, Br et I, et un sulfonate de formule -OSO₂R₉ où R₉ est choisi dans le groupe consistant en CF₃, un groupe alkyle (en C₁ à C₄), phényle, et phényle mono- ou disubstitué par un radical choisi parmi les groupes alkyle (en C₁ à C₄), un atome d'halogène et un groupe nitro; et W, V, V', R₁, R₂, R₃, R₄, R₅, R₆ et R₇ ont la même signification que celle définie ci-dessus pour le composé de formule (II),

6. Procédé selon la revendication 5, où le composé de formule (III) est le composé de formule (IIIa) où X a la même signification que dans la revendication 5:

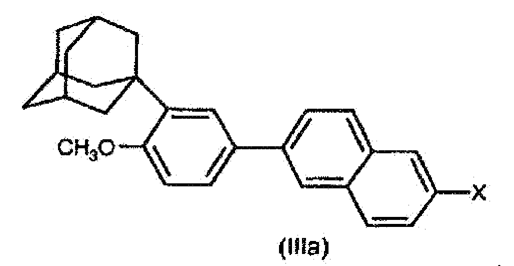

7. Le procédé selon la revendication 6, dans lequel l'atome d'halogène est Br.

8. Le procédé selon la revendication 7, dans lequel la réaction est réalisée avec du cyanure de cuivre.

9. Le procédé selon la revendication 6, dans lequel R₉ est CF₃.

10. Le procédé selon la revendication 9, dans lequel la réaction est réalisée avec du cyanure de zinc.

11. Le procédé selon l'une quelconque des revendications 9 et 10, dans lequel la réaction est réalisée en présence d'un catalyseur de palladium ou nickel.

12. Le procédé selon la revendication 11, dans lequel le catalyseur est un catalyseur de palladium.

13. Le composé de formule (X), dans lequel R₉ est un radical qui est choisi parmi un groupe CF₃, alkyle (en C₁ à C₄), phényle, et phényle qui est disubstitué par un radical alkyle (en C₁ à C₄):

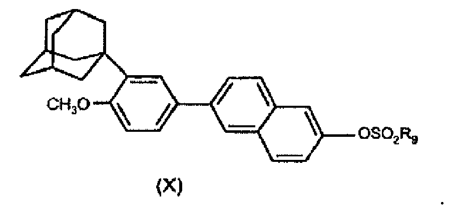

14. Le composé selon la revendication 13, dans lequel R₉ est -CF₃.

15. Le composé de formule (IX):
